# EUROPÄISCHE PATENTSCHRIFT

(11) **EP 0 895 758 B1**
(45) Veröffentlichungstag und Bekanntmachung des Hinweises auf die Patenterteilung: **04.08.2004**
(21) Anmeldenummer: 98108062.5
(22) Anmeldetag: 04.05.1998
(51) Int. Cl.: A61F 2/00, A61F 2/52

(54) **Brustprothese**
Breast prosthesis
Prothèse mammaire

(30) Priorität: 08.07.1997 DE 29712015 U
(43) Veröffentlichungstag der Anmeldung: 10.02.1999
(73) Patentinhaber: AMOENA Medizin-Orthopädie-Technik GmbH, 83064 Raubling (DE)
(72) Erfinder: Mulligan, Elisabeth, 83083 Riedering-Söllhuben (DE)
(74) Vertreter: Gossel, Hans K., Dipl.-Ing.

(56) Entgegenhaltungen:
- EP-A- 0 542 119
- EP-A- 0 588 756
- WO-A-94/16650
- CA-A- 2 101 509
- DE-U- 29 708 120
- FR-A- 2 293 187
- FR-A- 2 757 043

## Beschreibung

Die Erfindung betrifft eine Brustprothese, bestehend aus einem schalenförmigen Körper aus einer Masse von weich-elastischer Nachgiebigkeit, der in Kunststoffolien eingeschweißt ist, die dessen Außenseiten abdecken, wobei die Brustprothese auf ihrer Rückseite mit einer dauernd klebrig bleibenden Schicht oder im Abstand voneinander mit Bereichen mit dauernd klebrig bleibenden Schichten versehen ist.

Eine Brustprothese dieser Art ist beispielsweise aus EP 0 542 119 A1 bekannt. Hier besteht der schaenförmige Körper aus einer weich-elastisch eingestellten additionsvemetzenden Zweikomponenten-Silikon-Kautschukmasse. Diese bekannte Brustprothese hat sich als sogenannte selbsthaftende Prothese gut bewährt. Bei der bekannten Brustprothese läßt es sich allerdings nicht vermeiden, daß die Haftkraft der dauernd klebrig bleibenden Schicht mit der Zeit nachläßt.

Aufgabe der Erfindung ist es daher, eine Brustprothese der eingangs angegebenen Art zu schaffen, bei der die dauernd klebrig bleibende Schicht eine bessere Haftkraft über eine längere Zeit besitzt.

Erfindungsgemäß wird diese Aufgabe dadurch gelöst, daß die dauernd klebrig bleibende Schicht oder die dauernd klebrig bleibenden Schichten eine strukturierte Oberfläche mit Erhöhungen und/oder Vertiefungen aufweisen.

Überraschend hat sich gezeigt, daß die dauernd klebrige Schicht mit der in der erfindungsgemäßen Weise strukturierten Oberfläche eine bessere Haftkraft über eine längere Zeit besitzt.

Die Oberfläche der dauernd klebrig bleibenden Schicht kann mit Noppen versehen sein. Die Noppen können in einem vorgegebenen Raster, in Reihen oder auch ungleichmäßig verteilt angeordnet sein.

Nach einer anderen Ausführungsform ist vorgesehen, daß die Oberfläche mit näpfchenartigen Vertiefungen versehen ist, die ebenfalls in einem vorgegebenen Raster, in Reihen oder in beliebiger Verteilung angeordnet sein können.

Die Oberfläche kann auch mit Rippen versehen sein, die parallel zueinander verlaufen oder sich auch kreuzen können.

Die dauernd klebrig bleibende Schicht kann aus einer klebrig eingestellten additionsvernetzenden Zweikomponenten-Silikon-Kautschukmasse bestehen und sie kann weiterhin auf eine Tragfolie aufgebracht sein, die mit der die Prothese einhüllenden Folie verklebt oder verschweißt ist, wie es aus der EP 0 542 119 A1 bekannt ist.

Ausführungsbeispiele der Erfindung werden nachstehend anhand der Zeichnung näher erläutert. In dieser zeigen
- Fig. 1 bis Fig. 3: Rückansichten von Brustprothesen mit dauernd klebrig bleibenden Schichten mit strukturierten Oberflächen und
- Fig. 1a bis Fig. 3a: Schnitte durch die Randbereiche der Brustprothesen nach den Fig. 1 bis Fig. 3

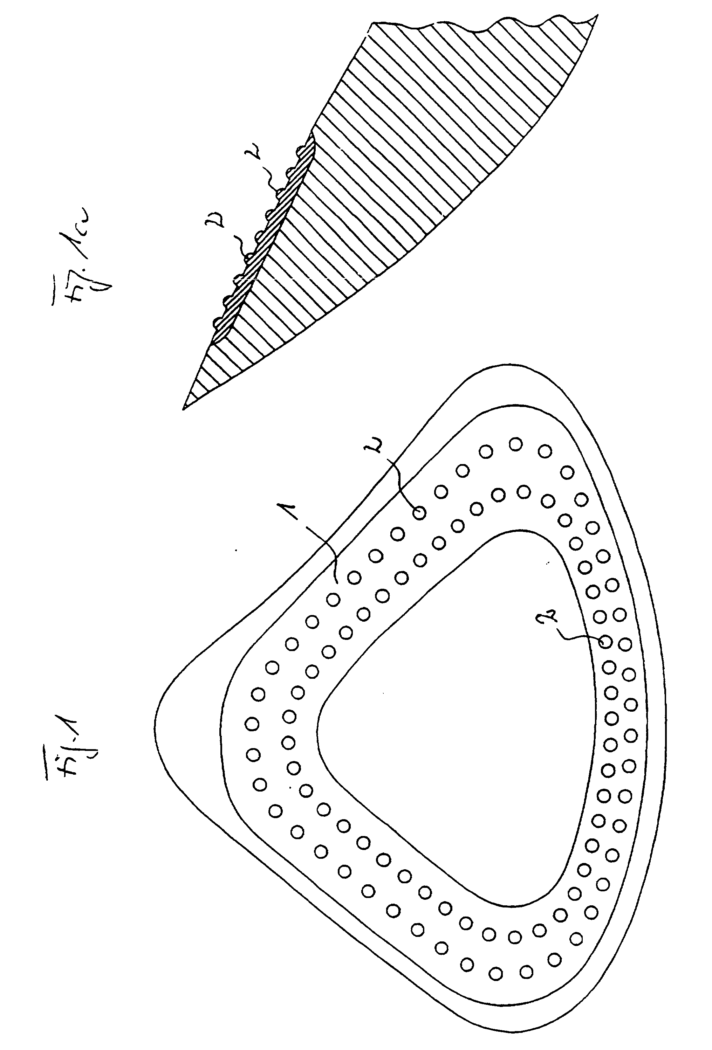

Aus den Fig. 1 bis 3 sind Rückansichten von Brustprothesen ersichtlich, die in ihrem Randbereich in einer Vertiefung mit dauernd klebrig bleibenden Schichten 1 versehen sind. Die Oberfläche der dauernd klebrig bleibenden Schicht nach Fig. 1 ist mit Noppen 2 mit abgerundeten Köpfen versehen.

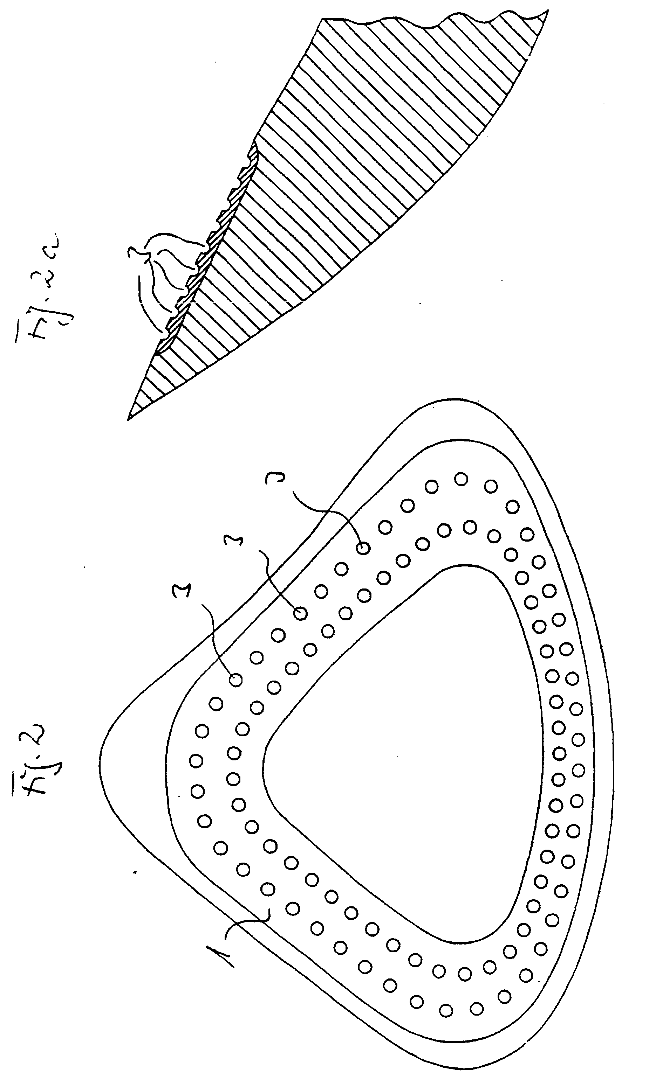

Bei der Ausführungsform nach Fig. 2 ist die dauernd klebrig bleibende Schicht näpfchenartigen Vertiefungen 3 versehen.

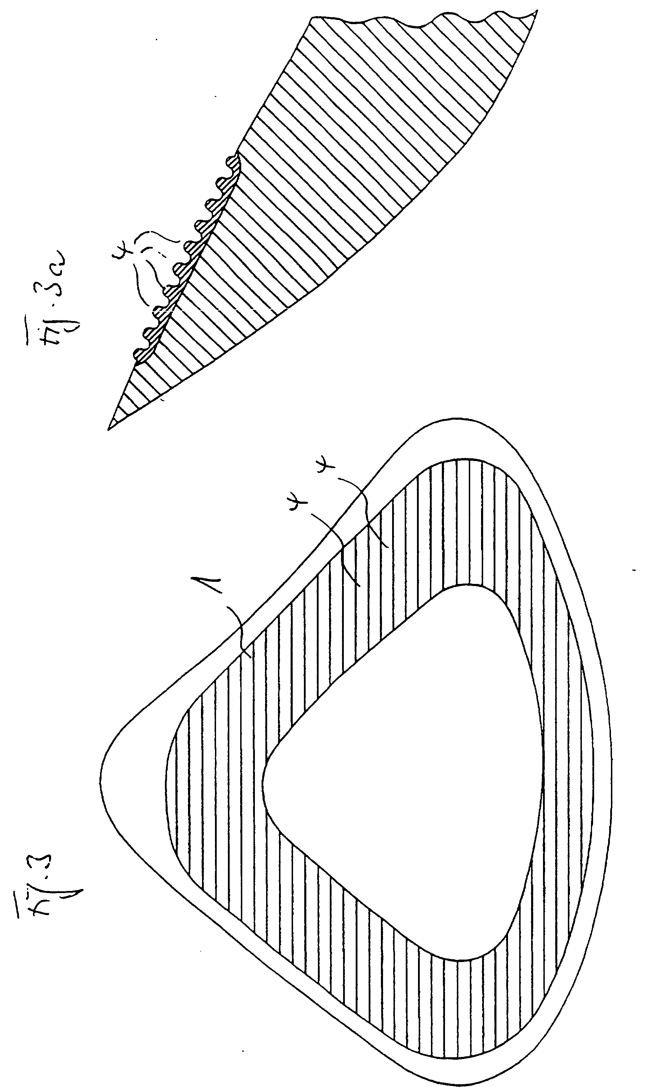

Bei der Ausführungsform nach Fig. 3 ist die dauernd klebrig bleibende Schicht mit zueinander parallelen rippenartigen Streifen 4 versehen.

## Patentansprüche

1. Brustprothese,
bestehend aus einem schalenförmigen Körper aus einer Masse von weich-elastischer Nachgiebigkeit, der in Kunststoffolien eingeschweißt ist, die dessen Außenseiten abdecken,
wobei die Brustprothese auf ihrer Rückseite mit einer dauernd klebrig bleibenden Schicht oder im Abstand voneinander mit Bereichen mit dauernd klebrig bleibenden Schichten versehen ist,
**dadurch gekennzeichnet,**
**daß** die dauernd klebrig bleibende Schicht (1) oder die dauernd klebrig bleibenden Schichten eine strukturierte Oberfläche mit Erhöhungen und/oder Vertiefungen aufweisen, so daß die Haftkraft über eine längere Zeit besser ist.

2. Brustprothese nach Anspruch 1, **dadurch gekennzeichnet, daß** die Oberfläche mit Noppen (2, 3) versehen ist.

3. Brustprothese nach Anspruch 1, **dadurch gekennzeichnet, daß** die Oberfläche mit näpfchenartigen Vertiefungen (3) versehen ist.

4. Brustprothese nach Anspruch 1, **dadurch gekennzeichnet, daß** die Oberfläche mit Rippen (4) versehen ist.

## Claims

1. Breast prosthesis,
consisting of a shell-shaped body which is made of a composition with soft resilient compliance and which is sealed in plastic films covering its outer sides,
the back of the breast prosthesis being provided with a permanently tacky layer or with areas of permanently tacky layers arranged at a distance from one another,
**characterized in that**
the permanently tacky layer (1) or the permanently tacky layers have a structured surface with elevations and/or depressions, so that the adhesive force is better for a longer time.

2. Breast prosthesis according to Claim 1, **characterized in that** the surface is provided with knobs (2, 3).

3. Breast prosthesis according to Claim 1, **characterized in that** the surface is provided with cup-like depressions (3).

4. Breast prosthesis according to Claim 1, **characterized in that** the surface is provided with ribs (4).

## Revendications

1. Prothèse mammaire,
constituée d'un corps en forme de coque en une masse apte à céder d'une manière molle et élastique, qui est scellé dans des feuilles de matériau synthétique, qui recouvrent les côtés extérieurs de celui-ci,
où la prothèse mammaire présente à son côté arrière une couche qui reste collante en permanence ou, espacées les unes des autres, des zones de couches qui restent collantes en permanence,
**caractérisée,**
**en ce que** la couche (1) qui reste collante en permanence ou les couches qui restent collantes en permanence présentent une surface structurée avec des surélévations et/ou creux de sorte que la force d'adhésion est meilleure pendant une plus longue durée.

2. Prothèse mammaire selon la revendication 1, **caractérisée en ce que** la surface est pourvue de tétons (2, 3).

3. Prothèse mammaire selon la revendication 1, **caractérisée en ce que** la surface est pourvue de creux (3) semblables à de petits godets.

4. Prothèse mammaire selon la revendication 1, **caractérisée en ce que** la surface est pourvue de nervures (4).
